# EUROPEAN PATENT APPLICATION

(11) **EP 2 535 711 A1**
(43) Date of publication of application: **19.12.2012**
(21) Application number: 11382199.5
(22) Date of filing: 15.06.2011
(51) Int. Cl.: G01N 33/50

(54) **Diagnostic marker for acute coronary syndrome**

(71) Applicant: Fundació Institut de Recerca Hospital Universitari Vall d'Hebron, Fundació Privada, 08035 Barcelona (ES); Ente Publico Hospital Universitario de Fuenlabrada, 28942 Fuenlabrada (ES)
(72) Inventor: Carcía Dorado, David, 08017 Barcelona (ES); Barba Vert, Ignasi, 08225 Terrassa (ES); Alonso Martín, Joaquín Jesús, 28023 Madrid (ES); Cristóbal Varela, Carmen, 28003 Madrid (ES)
(74) Representative: ZBM Patents

(57) **Abstract**

The invention discloses an *in vitro* method for diagnosing acute coronary syndrome in a patient. The method is performed in blood and uses means to detect selective compounds.

## Description

The present invention provides methods and markers for the diagnosis and prognosis of Acute Coronary Syndrome (ACS). The invention is embedded in the field of medicine at ambulatory and emergencies levels.

### BACKGROUND ART

Acute Coronary Syndrome (ACS) is a clinical situation characterized by the occurrence of myocardial ischemia (insufficient coronary flow causing dysfunctional and metabolic changes) secondary to acute total or sub-total coronary obstructions. The coronary arteries are the arteries supplying blood to the cardiac muscle (myocardium). The acute reduction in blood flow in the cardiac muscle is a characteristic feature of ACS that differentiate it from stable angina, in which myocardial ischemia does not occur as a consequence of coronary flow reduction, but instead as the consequence of an increase in the metabolic demands of myocardium, usually as the consequence of physical exercise. The acute reduction in coronary blood flow causing ACS is usually due to a thrombus on an active, complicated atheroma plaque, which is narrowing a coronary arterial lumen. Active and complicated means that occurs when there is a disruption of the plaque surface in contact with blood due to a tear or ulcer in its endothelial layer that exposes blood to the prothrombotic materials present in the atherosclerotic lesion. The thrombus is mainly formed by platelets and fibrine, and acutely reduces or blocks the lumen of the coronary artery causing myocardial ischemia. There are also other mechanisms involved in the lowering of the cardiac muscle flow, such as the coronary artery constriction or the vessel spasm, which may be also induced by substances released by the platelets contained in the intracoronary thrombus that stimulate the contraction of the smooth muscle cells present in the vascular wall. The severity of ACS depends on the magnitude and duration of the flow reduction. In case the ischemia is sever and prolonged, the cells of the cardiac muscle may die. According to its severity ACS may cause three different clinical presentations: unstable angina, non Q acute myocardial infarction and Q-wave, or transmural, acute myocardial infarction. When cell dead is not observed, it is said that ACS is recognized as unstable angina (UA). On the contrary, if cell dead is observed, then the disease is called myocardium infarction (MI) that may cause Q waves in the ECG if it involves most of myocardial wall thickness at the infarct site (Q wave infarct) or may not cause Q waves indicating that necrosis involves only a minor fraction of the ventricular wall (non Q-wave myocardial infarction).

Typical symptom of ACS is an acute thoracic pain, said pain usually radiating to left arm or both arms. Patients with unstable angina may have or not transient changes in the ST segment of the ECG, but serial blood samples over 24 hours show no or minimal increases in markers of necrosis (substances coming from death myocytes with broken cell membranes). Patients with non-Q wave myocardial infarction may show no ECG changes, or changes similar to those in patients with unstable angina, but show markers of cell death during the 24 hours after symptoms onset. Patients with Q-wave myocardial infarction usually present ECG changes different to those observed in patients with unstable angina or non-Q infarcts. These ECG changes in case of Q-wave myocardial infarction use to consist in elevation of the ST segment, and Q waves appear during the following hours. They also show markers of cardiomyocyte cell death during the 24 hours after symptoms onset.

The prognosis is more severe in patients with Q wave than in those with non-Q wave infarcts and more severe in non-Q wave infarcts that in patients with unstable angina. Among those with unstable angina, those with ECG changes or with tiny increases in markers of necrosis not reaching the magnitude necessary for the diagnosis of infarction have a worse prognosis than those with none of these changes. However, even patients with unstable angina without ECG changes or markers of necrosis have an increased risk of serious complications during the following hours and days, which make necessary a close monitoring

Early and efficient detection of ACS is of great importance since the prognostic of the subject suffering of ACS is really severe, potentially leading to death. At the same time, a fast and efficient identification of the low risk patients that can be ambulatory treated is also important due to economical reasons.

Initial diagnostic of ACS is based on the patient (subject) clinical report (if any), on the performance of an electrocardiogram and on the detection of blood cardiac biomarker levels. The biomarker levels most widely used are the Creatine Kinase-myocardial band (CK-MB) and the Cardiac Troponin.

Additionally, other image tests are performed, such as echocardiographies and coronary arteriography, as well as effort tests.

An example of the typical and currently determined biomarkers for the diagnosis of ACS is disclosed in Moe et al., "Current Trends in Diagnostic Biomarkers of Acute Coronary Syndrome", Annals Academy Medicine Singapore - 2010, Vol. 39, pp. 210-215. This document mentions as widely used ACS biomarkers the Cardiac Troponin, Creatine Kinase-myocardial band (CK-MB), B-type Natriuretic Peptides (BNP) and Myoglobin. As emerging biomarkers there are cited the biomarkers of oxidative stress (lipoprotein-associated phospholipase A2), and biomarkers of tissue necrosis (fatty acids binding proteins-FSBPs). Moe et al. indicate that however, no available biomarker offers ideal diagnostic properties for ACS, such as early detection, high sensitivity and specificity, easy availability, and cost effectiveness.

A great number of subjects suffering from ACS neither have electrocardiography nor analytical abnormalities, including CK-MB and Cardiac Troponin, but behave an elevated risk of developing severe ischemic complications hours or days after the onset of ACS.

In an attempt of providing usable biomarkers for detecting another disease type, the coronary artery disease (CAD), the US patent application US20100173346 discloses a group of biomarkers that may be selected with the aim of varying the specificity and sensitivity of a test to determine if a patient is suffering from any form of CAD. Different technologies are cited, such as the ¹H-NMR, or some colorimetric assays. The document also discloses a method for assessing cardiovascular disease status, specifically of a CAD. The method comprises determining in a patient with a high probability of suffering a cardiovascular disease, the levels of several metabolites.

It is important to emphasize the difference between stable angina and ACS. Stable angina deteriorates the quality of life of patients by limiting their exercise capacity, but has little effect on prognosis and gives time to perform interventions to modify both. On the contrary, ACS denotes an unstable situation in which ischemia occurs generally at rest, can worsen very rapidly in hours or minutes, and can cause myocardial necrosis, cardiac failure, arrhythmias and death.

Nowadays, the technology known as ¹H-NMR-based metabonomics is proposed as a rapid methodology for diagnosing coronary heart disease. This technology is disclosed in Brindle et al., "Rapid and noninvasive diagnosis of the presence and severity of coronary heart disease using 1H-NNMR-based metabonomics", Nature Medicine -2002, Vol. 8 (12), pp. 1439-1444, wherein it is indicated that proton nuclear magnetic resonance may be used as diagnostic method by metabonomic analysis in plasma. The technology analyses all those compounds (metabolites) which are present in a body fluid sample and which may be detected by nuclear magnetic resonance.

Apart of relating to stable anginas, which are different in etiology and consequences than ACS, the teaching of Brindle et al. *(supra)* is subjected to some contradictory statements indicating that this spectroscopic tool is not reliable at all. Indeed, as stated by Kirschenlohr et al., "Proton NMR analysis of plasma is a weak predictor of coronary artery disease", Nature Medicine - 2006, Vol. 12 (6), pp. 705-709, the data obtainable by ¹H-NNMR-based metabonomics may be altered depending on the genus of the patient and the previously administration of statins, a class of drug used to lower cholesterol to prevent cardiovascular diseases.

The detection in a true and efficient manner of ACS in a subject, preferably performed at ambulatory or emergency level is a real necessity of the art. Considering the above-mentioned scenario and, in spite of the efforts made, there is still the need in the state of the art of further markers for detecting ACS.

### SUMMARY OF THE INVENTION

With the aim of solving the problems mentioned before, the inventors performed a deep study of the body fluid metabolites detectable in a body fluid sample in subjects which were previously diagnosed of acute coronary syndrome (ACS). Although the rationale of the nowadays applied ¹H-NMR-based metabonomics technology, leads the skilled man to observe the aliphatic part of the spectra, the inventors surprisingly found in the aromatic region of the spectra that formate (conjugated base of formic acid) could be differentially detected in cases of ACS.

Thus, in a first aspect the invention relates to an *in vitro* method for diagnosing acute coronary syndrome (ACS) in a subject suspected of suffering it, comprising the step of detecting the presence of formic acid or a salt thereof in an isolated sample of said subject, and wherein the detection of the formic acid is indicative of acute coronary syndrome.

The detection of formic acid or formate (the conjugated base of formic acid) allows the control of all the patients, minimizing the risk of underestimating subjects with ACS. Thus, the method avoids the discrimination of subjects being false negative subjects, and at the same time avoids the admission of subjects not suffering from ACS, which represent a great cost for the sanitary system.

As will be illustrated below the detection of formic acid or formate represents a tool leading to significant results, which are independent of the subject genus and of other parameters. This is of great importance, since the method may be easily standardized.

Moreover, the detection of such a metabolite can be performed early (when the subject enters and is suspected of ACS) by easy obtaining a body fluid sample (i.e.: serum). Further, the detection of formic acid or formate (when forming part of a physiological salt) has a high sensitivity and specificity and, further, it is cost effectiveness.

Once diagnosis is performed, different prognostics are involved due to the implicit variability of ACS.

In a second aspect, the invention relates to a method of deciding or recommending whether to initiate pharmacological treatment of a subject suspected of suffering acute coronary syndrome, which method comprises the steps of:
a) detecting, *in vitro,* the presence of formic acid or a salt thereof in an isolated sample of the subject; and
b) diagnosing acute coronary syndrome if formic acid or a salt thereof is/are detected;
wherein:
if the subject is diagnosed of acute coronary syndrome, then it is recommended the initiation of a therapy selected from the group consisting of double antiplatelet therapy and anticoagulation, and nitroglycerin perfusion; or
if the patient is diagnosed of not suffering acute coronary syndrome, other causes of acute chest pain are checked.

Emphasis is added in that ruling out ACS may also allow early discharge, which represents a large clinical and economical advantage.

A third aspect of the invention is the use of means for detecting the presence of formic acid or a salt thereof in an isolated sample for the diagnosis of acute coronary syndrome in any of the methods of the above-mentioned aspects of the invention.

In a fourth aspect, the invention aims the use of formic acid or a salt thereof as clinical diagnostic marker of acute coronary syndrome.

### BRIEF DESCRIPTION OF THE DRAWINGS

FIG. 1, related to Example 1, represents a discriminant analysis score plot (PLS-DA) of the two components t[1] and t[2] of a model statistically (p=0,01) able to differentiate between acute and chronic phase patients from set 1. Each dot corresponds to an individual patient. Square dots correspond to samples obtained at the acute phase, 72 hours after the onset of chest pain while round dots correspond to samples obtained from the same patients 6 months after the main ACS event. A means Acute; C means Chronic.
FIG. 2, related to Example 1, is the ¹H-NMR spectra of a typical serum sample before (A) and after (B) the addition of formic acid. Formic acid co-resonates with the peak at 8.45 ppm thus, showing unambiguous assignation to formic. X axis is shown in ppm, Y axis in arbitrary units.
FIG. 3, also related to Example 1, is a graph showing the area of the formate peak for acute and chronic patients in set 1. A means Acute; C means Chronic; and F is formate. Data are in arbitrary units.

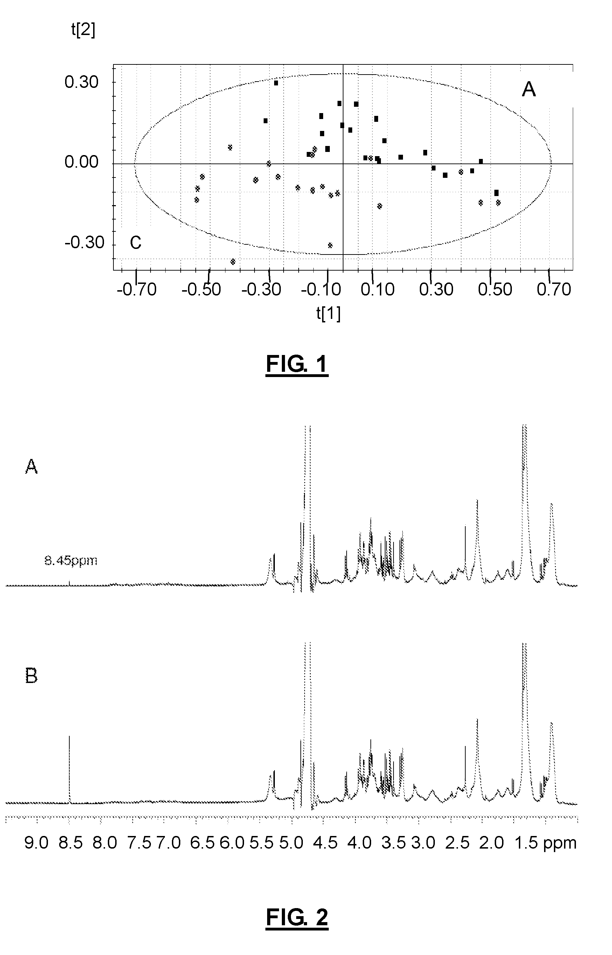

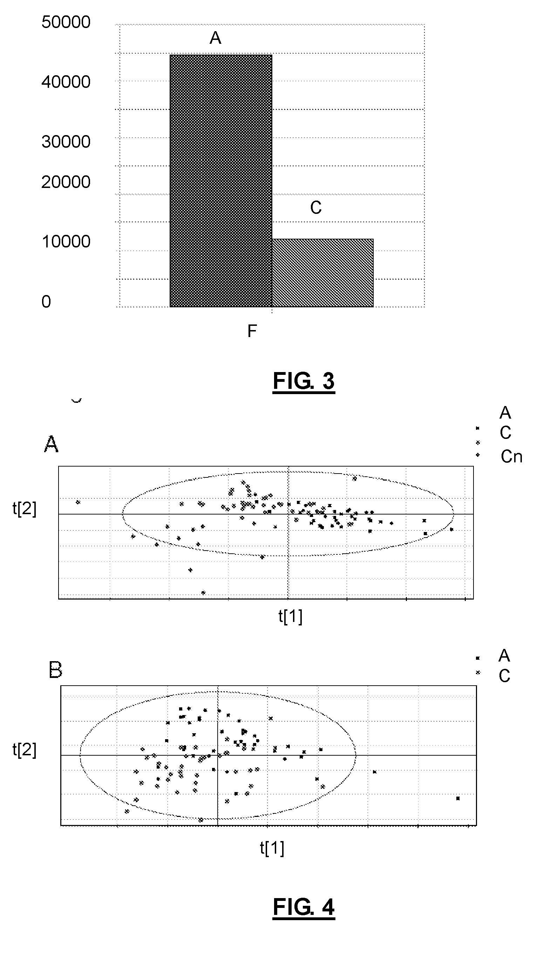

FIG. 4, related to Example 2, is a discriminant analysis (PLS-DA) in patient set 2. Panel (A) corresponds to a three group model (Acute, Chronic, Control) and panel (B) to a two group model (Acute, Chronic) as in FIG. 1 each dot corresponds to an individual sample. Square dots correspond to samples obtained in the acute phase (within 72 hours of the onset of pain). Round dots correspond to samples obtained after 6 months of ACS and rhombus correspond to samples obtained from patients admitted to accident and emergency during hospital admission with a diagnosis different from ACS. A means Acute; C means Chronic; Cn is Control; and F is formate.
FIG. 5, related to Example 2, is a graph showing the area of the formate peak for acute, chronic and control patients in set 2. A means Acute; C means Chronic; and Cn is Control. Data are in arbitrary units.
FIG. 6, relating to example 2, represents the Receiver Operating Characteristic (ROC) for the discrimination between Acute and Chronic samples in patient of set 2. (S) is sensitivity; (100-Sp) is the specificity.
FIG. 7, related to Example 3, is a graph showing the examples of formic acid detection by following the absorbance at 340 nm (Ab 340 nm; Y-axis). Graphs corresponding to two samples (one chronic, C; one acute, A) are shown. The slope of the curve is proportional to the amount of formic acid in the sample. T is time in seconds (X-axis).

### DETAILED DESCRIPTION OF THE INVENTION

The *in vitro* method according to the invention comprises the step of detecting the presence of formic acid or a salt thereof in an isolated sample of the subject suspected of suffering ACS. As will be illustrated below by means of examples, the mere presence of the compound, detected by several different means is indicative of the syndrome.

In a preferred embodiment of the first aspect of the invention, the method comprises detecting acute coronary syndrome which is associated to a disease selected from the group consisting of unstable angina (UA), non-Q wave myocardial infarction (non-Q wave), and Q wave myocardial infarction (Q wave). As above indicated, ACS may be due to one of these three diseases, directly or indirectly if other primary cause finally induces unstable angina (UA), non-Q wave myocardial infarction (non-Q wave) or Q wave myocardial infarction (Q wave).

Once the formic acid is detected, it is used to screen the subject and, if considered necessary, the amount of the biomarker is determined. Independently of the amount determination, the sample is compared with a standard, thus allowing the classification of the subject. This standard can be obtained from a known normal person. The known normal person is a healthy person. Alternatively, the method may use as standard the value of formic acid from an ACS suffering patient, or both.

For the performance of the method, any isolated sample which is preferably a bodily fluid, may be used. Thus, in a preferred embodiment of the first aspect of the invention, the isolated sample (test) is selected from the group consisting of whole blood, serum, plasma, urine and saliva.

When a blood sample is used, the sample may be the whole blood or the blood previously treated to obtain the plasma or the serum, both also equally useful. In a most preferred embodiment, the isolated sample is blood serum.

Also blood serum is also preferred as isolated sample for the method of deciding or recommending whether to initiate pharmacological treatment of a subject suspected of suffering acute coronary syndrome, according to the second aspect of the invention. This is so, since blood extraction is a common practice yet when a subject is suspected of ACS.

The specificity of the biomolecule (formic), in combination with the current practice of blood extraction and manipulation makes of the methods of the invention a rapid way to diagnose the onset of ACS. This is of great relevance due to the bad prognostic of the disease, as above-exposed.

In a preferred embodiment of the method of deciding or recommending whether to initiate pharmacological treatment of a subject suspected of suffering acute coronary syndrome, if the subject is diagnosed of acute coronary syndrome, the further therapeutic interventions selected from the group consisting of an invasive coronarography, a percutaneous coronary intervention, and a surgical coronary revascularization are advised.

In another preferred embodiment of the method of deciding or recommending whether to initiate pharmacological treatment of a subject suspected of suffering acute coronary syndrome, if the subject is diagnosed of not suffering acute coronary syndrome, the other causes of acute chest pain checked are selected from the group consisting of pulmonary thromboembolism and acute aortic diseases.

In another preferred embodiment of the method of deciding or recommending whether to initiate pharmacological treatment of a subject suspected of suffering acute coronary syndrome, deciding or recommending whether to initiate pharmacological treatment is optionally made in consideration of the result of an examination of the patient by a cardiologist.

All these causes of acute chest pain may be preferably checked by using image techniques like computed axial tomography scan (CT scan) or echocardiography, among others.

The *in vitro* method of the invention is performed, preferably, by detecting formic acid in the sample by means of a spectroscopic assay.

Previously or simultaneously to the detection of the formic acid or salts thereof, the components integrating the sample may be resolved or separated. Depending on the spectroscopic assay used, separation of the components integrating the mixture, or manipulation of the sample is not needed, and the compound of interest is directly detected.

Preferred spectroscopic assays used for detecting the presence of formate or formic acid in a test sample for the diagnosis of ACS include proton nuclear magnetic resonance (¹H-NMR), as well as a colorimetric assay (or any equivalent optical spectroscopy assay).

¹H-NMR is a spectroscopic assay that may be used both to resolve a complex mixture and to detect what metabolites or compounds are present in a sample. ¹H-NMR is advantageously applicable since it does not require the previous manipulation of the samples.

A colorimetric assay (or analysis) is a quantitative chemical analysis that measures the colour intensity produced by reaction of the sample with a reactant. The reagents are provided to produce detectable colour in the tested sample. In other words, a colorimetric analysis is a method of determining the concentration of a chemical element or chemical compound in a solution with the aid of a colour reagent. It is applicable to both organic compounds and inorganic compounds and may be used with or without an enzymatic stage. The method is widely used in medical laboratories and for industrial purposes, e.g. the analysis of water samples in connection with industrial water treatment.

Thus, a marker (formic acid or a salt thereof) can be detected in a complex sample and/or quantified using several of the available separation methods. Examples of separation methods include mass spectrometry methods, such as electrospray ionization mass spectrometry (ESI-MS), matrix-assisted laser desorption ionization time-of-flight mass spectrometry (MALDI-TOF-MS), desorption/ionization on silicon (DIOS), Fourier transform mass spectrometry (FTMS). Spectrometric techniques include also resonance spectroscopy and optical spectroscopy.

Other suitable separation methods include chemical extraction techniques, chromatography, such as ion exchanges column, affinity column, molecular exclusion column, gel electrophoresis (one-dimensional or two-dimensional), thin-layer chromatography, etc. All these separation techniques may be performed in tandem, for example by coupling mass spectroscopy with liquid chromatography.

In a preferred embodiment of the first and second aspects of the invention said spectroscopic means for detecting formic acid or a salt thereof comprise a pair of enzyme/coenzyme, wherein the enzyme is able to oxidize the formic acid to carbon dioxide and wherein the coenzyme is reduced. A preferred example of the pair enzyme/coenzyme includes a formate dehydrogenase and the coenzyme Nicotinamide adenine dinucleotide (NAD+). NAD+ is reduced to NADH, and formate is oxidized to carbon dioxide.

This is an example of optical spectroscopy assay in which the detection of formate is performed enzymatically by oxidization of formate to CO₂ with the enzyme formate dehydrogenase (EC-1.2.1.2) followed by the reduction of NAD to NADH, which can be spectrophotometrically followed at 340 nm (UV light spectra). Other suitable assays may be those visually detectable (Visible light spectra) without the use of a spectrophotometer, i.e., a colorimetric assay visually detectable.

The pair enzyme/coenzyme may form part of a kit, wherein all the components needed to perform the reaction (additives, solvents, etc.) are distributed together. On the alternative, the means used for detecting the presence of formic acid or its physiological salts for the purpose of the invention may also be integrants or reagents of different commercial kits.

Additionally, dipsticks or equivalent thin strips of plastic may be used to detect the presence of the marker (formic/formate) in the sample. These strips are examples of colorimetric assays that change of colour when specific substances are present in the sample.

As will be illustrated in the following examples, the formate or formic acid may be used as a specific, highly sensitive clinical diagnostic marker for ACS, and preferably it is detected in a serum sample.

For the purposive of understanding, the following definitions are included.

"A physiological salt of formic acid" refers to any salt that can be found in the sample, wherein the negative anion is the formate (conjugated base of formic acid. The detection in a sample of formic acid or of formate depends on the employed methodology, which can require the previously manipulation of the sample.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Sample source and Methods

Serum samples were obtained from 119 patients from "Hospital Universitario de Fuenlabrada", Madrid, Spain; all of them gave their informed consent and the study was approved by the ethics committee of "Hospital Universitario de Fuenlabrada".

### Example 1

Sample Set 1 consisted of 56 samples corresponding to 23 patients admitted at the hospital with the diagnosis of ACS (n=20 UA, n=12 non-Q wave, n=14 Q wave): one sample was obtained within the first 72h after the onset of chest pain (acute phase) and another sample after 6 months from the ACS (chronic phase).

¹H-NMR spectroscopy was performed as previously described in Barba et al., "Nuclear magnetic resonance-based metabolomics predicts exercise-induced ischemia in patients with suspected coronary artery disease", Magn. Reson. Med. 2008; Vol. 60, pp.:27-32. Briefly, 200 µl of serum were mixed with 400 µl of phosphate-buffered saline (PBS) prepared with deuterium oxide. Spectra were acquired at 30°C on a 400 MHz vertical bore magnet interfaced to a BrukerAvance 400 console and consisted in the accumulation of 64 scans with a CPMG pulse sequence with an effective T2 delay of 32 milliseconds (ms).

Peak identification was based on chemical shift from 1 and 2D (both ¹H-¹H and ¹H-¹³C) spectra by comparison to data public aces data (Human metabolome project). Unambiguous identification was accomplished by spiking standards into selected samples.

Pattern recognition: Spectra were integrated between 0.5 and 9 ppm in 800 bins, the output vector representing each spectrum was normalized across the integral regions excluding the water resonance between 4.5 and 5.25 ppm. Datasets were imported into SIMCA-P software (Umetrics, Umea, Sweden) and preprocessed using pareto scaling of each variable to (1/*Sₖ*)^{1/2}, where *Sₖ* is the standard deviation of the variable (integral region) *k*.

Data were analyzed using principal component analysis.

Principal Component Analysis (PCA) is a quantitatively rigorous method for replacing a group of variables with a smaller number of variables called principal components (PC), which are linear combinations of the original variables. All principal components are orthogonal to each other so there is no redundant information. Projecting the observations on one of these axes generates another new variable designed to maximize the description of the variance in the data set. PCA is used to examine trends and clustering in an unsupervised manner regardless of the group/pathology of each sample.

When PCA proved inadequate to define clustering, a supervised approach, (discriminant analysis) a Partial least squares Discriminant Analysis (PLS-DA) was used.

PLS-DA is a generalization of PCA where a projection model is developed predicting class membership from the variables via scores of these variables through a generalized multiple regression method that can cope with a number of variables being correlated with class membership.

In this type of analysis models are created in order to minimize intergroup while maximizing intragroup variation.

As can be seen in FIG. 1, it was possible to differentiate spectra from patient in set 1 using supervised pattern recognition analysis (PLS-DA analysis gave a 2 component model R²X=0,575; R²Y=0,56; Q²=0,412, where R²X, R²Y correspond to the goodness of fit for the X and Y variables, respectively, and Q² to the goodness of prediction or the predicted variation). The metabolites responsible for the differences were lipids and glucose. A similar analysis using only the aromatic part of the spectra after removing effects from lipids and glucose gave as a result an improved model (PLS-DA analysis gave a 6 component model R²X=0,703; R²Y=0,968; Q²=0,679). The most relevant peak in this later analysis appeared at 8,45 ppm and was assigned to formate by comparison to published databases; it was later unambiguously assigned to formate by adding the pure compound to selected samples. FIG. 2 shows the ¹H-NMR spectra of a typical serum sample before (A) and after (B) the addition of formic acid.

As depicted in FIG. 3, which is a bar diagram showing the area of the formate peak for acute (A) and chronic (C) patients in set 1, the measure of the formate peak showed relevant differences between acute and chronic samples (peak area in arbitrary units presented as mean 44709 ± 950 in Acute patients vs 12031 ± 307 in Chronic patients; p=1,4*10⁻⁷).

### Example 2.

Set 2 consisted of 96 unpaired serum samples corresponding to 48 patients within the acute phase of the ACS (n=20 UA, n=12 non-Q wave, n=14 Q wave), 36 patients at the chronic phase, after 6 months from ACS (n=13 UA, n=11 non-Q wave, n=12 Q wave) and a group of 12 controls (average matched for sex and age) from patients admitted at the emergency room with a diagnosis different from ACS.

¹H-NMR spectroscopy and pattern recognition as in Example 1 were performed.

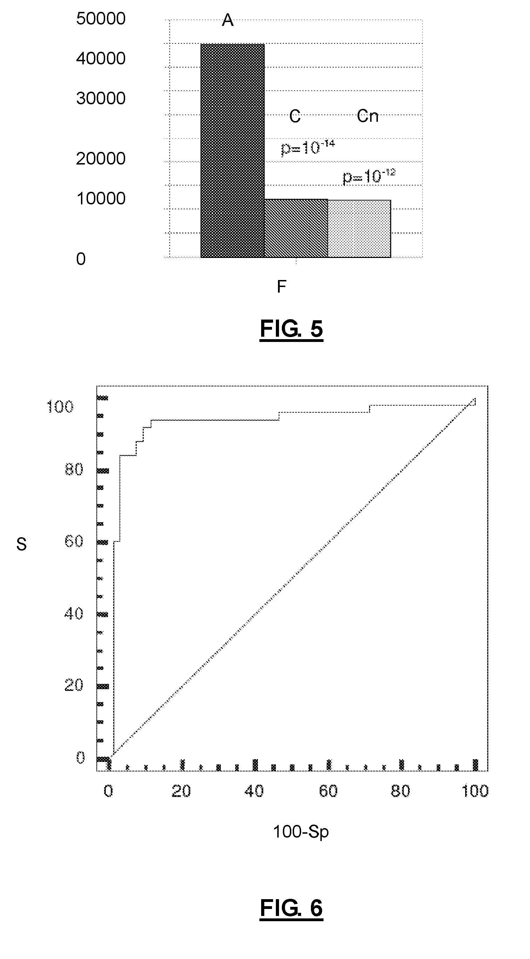

Similar results to those of Example 1 were obtained for sample set 2, a PLS-DA model differentiated between acute and chronic samples using the whole spectra (3 components, R²X=0,728; R²Y=0,616; Q²=0,469) lipids and glucose being the most relevant metabolites in the differentiation. FIG. 4 shows the results of the discriminant analysis in patient set 2. Panel (A) corresponds to a three group model (Acute, Chronic, Control) and panel (B) to a two group model (Acute, Chronic), and as in FIG. 1 each dot corresponds to an individual sample. As in the case of set 1, once the analysis was performed on the aromatic part of the spectra and the contribution of lipids and glucose was removed, formate was the most important metabolite in the model. FIG. 5, a graph showing the area of the formate peak for acute, chronic and control patients in set 2, shows the amount of formate obtained in acute, chronic and control patients which were different between acute, chronic and control patients (122935 ± 8690; 41630 ± 12277; 33730 ± 14922; p=7*10⁻⁹ between acute and chronic and p=1*10⁻⁴ between acute and control, measures in arbitrary units).

ROC (Receiver Operating Characteristic) curve analysis, depicted in FIG. 6, shows that formate is a good biomarker for the presence of acute coronary syndrome, since it reveals a great specificity accompanied with a great sensitivity.

In a ROC analysis the sensitivity and specificity for each specified value (in this case for the formic acid value) is represented. When the values follow the diagonal line in a ROC curve, it is said that the predictive factor is equal to random. On the contrary, if the values are near the coordinates (0,1), this means that a good predictive factor is achieved.

### Example 3. Spectrophotometric determination of formic acid in serum.

It is also possible to determine the presence of formic acid in serum using a colorimetric analysis. Serum samples were analyzed using and enzymatic reaction with the enzyme formate dehydrogenase (EC-1.2.1.2) from *Candida boidinii.* If formic acid was present in the sample it was oxidized to CO₂ with the enzyme formate dehydrogenase (EC-1.2.1.2). This reaction was coupled to the reduction of NAD+ to NADH, which can be spectrophotometrically followed at 340 nm.

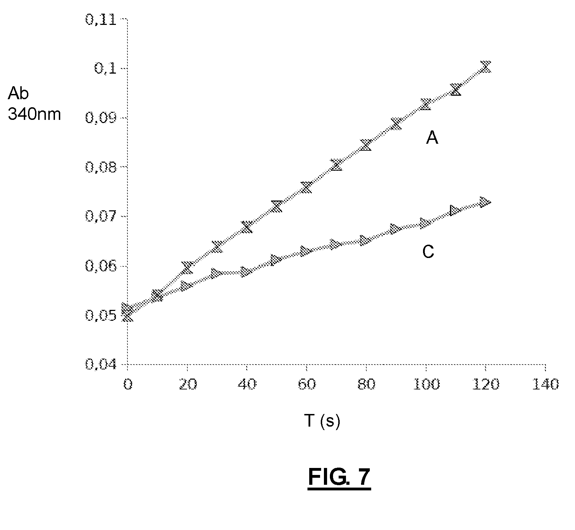

Formic acid was measured as increase of absorbance at 340 nm over 2 minutes from 200 µl of serum in a final volume of 500 µl with phosphate buffer saline solution (PBS) at pH 7.2, room temperature (18°C-25°C) containing 1 unit of formate dehydrogenase and 1 mM NAD+. FIG. 7 shows examples of formic acid spectrophotometric measures. As can be deduced from the graphic in samples of ACS patients (A), the absorbance units are greater than those of the chronic (C) patients, and the slope (Absorbance units / time unit) is greater in acute than in chronic patients.

All these examples and data allow considering formic acid or a salt thereof (formate) a good biomarker for ACS. The detection allows minimizing the false positive rate, as well as the false negative subjects. Additionally, the detection of this compound may be performed in a simple way by using colorimetric assays, which can be applied at ambulatory or emergency level.

Moreover, in case the center where the subject suspected of suffering ACS is conducted has all the instrumental needed to perform a ¹H-NMR spectroscopy analysis, the technology allows a feasible and also fast method to discriminate between ACS or any other disease.

The detection of formic acid is then a sensitive and specific way to diagnose ACS in an improved way.

### REFERENCES CITED IN THE APPLICATION

- Moe et al., "Current Trends in Diagnostic Biomarkers of Acute Coronary Syndrome", Annals Academy Medicine Singapore - 2010, Vol. 39, pp. 210-215.
- US20100173346.
- Brindle et al., "Rapid and noninvasive diagnosis of the presence and severity of coronary heart disease using 1H-NMR-based metabonomics", Nature Medicine -2002, Vol. 8 (12), pp. 1439-1444.
- Kirschenlohr et al., "Proton NMR analysis of plasma is a weak predictor of coronary artery disease", Nature Medicine -2006, Vol. 12 (6), pp. 705-709.
- Barba et al., "Nuclear magnetic resonance-based metabolomics predicts exercise-induced ischemia in patients with suspected coronary artery disease", Magn Reson Med. 2008; Vol. 60, pp.:27-32.

## Claims

1. An *in vitro* method for the diagnosis of acute coronary syndrome (ACS) in a subject suspected of suffering it, comprising the step of detecting the presence of formic acid or a salt thereof in an isolated sample of said subject, wherein the detection of the formic acid is indicative of acute coronary syndrome.

2. The *in vitro* method of claim 1 wherein acute coronary syndrome is associated to a disease selected from the group consisting of unstable angina (UA), non-Q wave myocardial infarction (non-Q wave), and Q wave myocardial infarction (Q wave).

3. The in *vitro* method according to any of claims 1-2, wherein the isolated sample is selected from the group consisting of whole blood, blood serum, plasma, urine, and saliva.

4. The *in vitro* method of claim 3, wherein the isolated sample is blood serum.

5. A method of deciding or recommending whether to initiate pharmacological treatment of a subject suspected of suffering acute coronary syndrome, which method comprises the steps of:
a) detecting, *in vitro,* the presence of formic acid or a salt thereof in an isolated sample of the subject; and
b) diagnosing acute coronary syndrome if formic acid or a salt thereof is/are detected;
wherein:
if the subject is diagnosed of acute coronary syndrome, then it is recommended the initiation of a therapy selected from the group consisting of double antiplatelet therapy and anticoagulation, and nitroglycerin perfusion; or if the patient is diagnosed of not suffering acute coronary syndrome, other causes of acute chest pain are checked.

6. The method of claim 5, wherein the isolated sample is serum.

7. The *in vitro* method according to any of claims 5-6, wherein if the subject is diagnosed of acute coronary syndrome, further therapeutic interventions selected from the group consisting of an invasive coronarography, a percutaneous coronary intervention, and a surgical coronary revascularization are advised.

8. The *in vitro* method according to any of claims 5-6, wherein if the patient is diagnosed of not suffering acute coronary syndrome, the other causes of acute chest pain checked are selected from the group consisting of pulmonary thromboembolism and acute aortic diseases.

9. The in *vitro* method according to any of claims 1-8, wherein the detection is carried out be means of an spectroscopic assay.

10. Use of means for detecting the presence of formic acid or a salt thereof in an isolated sample for the diagnosis of acute coronary syndrome in any of the methods of claims 1 to 9.

11. The use of claim 10, wherein said means comprise a pair of enzyme/coenzyme, wherein the enzyme is able to oxidize the formic acid to carbon dioxide and the coenzyme is reduced.

12. The use of claim 11, wherein the pair enzyme/coenzyme is a formate dehydrogenase and the coenzyme is NAD.

13. The use of any of claims 10-12, wherein the pair enzyme/coenzyme forms part of a kit.

14. Use of formic acid or a salt thereof as clinical diagnostic marker of acute coronary syndrome.

15. The use of claim 14, as clinical diagnostic marker in a serum sample.
